# EUROPEAN PATENT APPLICATION

(11) **EP 4 725 392 A2**
(43) Date of publication of application: **15.04.2026**
(21) Application number: 25207160.0
(22) Date of filing: 07.10.2025
(51) Int. Cl.: A61B 3/00, A61B 3/10, A61B 3/103

(54) **COMPUTER IMPLEMENTED APPLICATION PROGRAM FOR MYOPIA MANAGEMENT SERVICE**

(30) Priority: 08.10.2024 JP 2024176658
(71) Applicant: Toho University, Tokyo 143-8540 (JP); MENICON CO., LTD., Naka-ku Nagoya-shi, Aichi 460-0006 (JP)
(72) Inventor: MATSUMURA, Saiko, Tokyo, 143-8540 (JP); KATO, Hideki, Nagoya-shi, Aichi 460-0006 (JP); NOMURA, Hiroko, Nagoya-shi, Aichi 460-0006 (JP); ASAI, Hikaru, Nagoya-shi, Aichi 460-0006 (JP)
(74) Representative: Winter, Brandl - Partnerschaft mbB

(57) **Abstract**

An application program (30a) for a user performing processes in a user terminal device (12) including a display part (20), the user terminal device (12) being used by the user who is a target of a myopia management service, the processes including: a measurement acquisition process acquiring a measurement of an ocular axial length of the user; and a measurement display process displaying the measurement acquired by the measurement acquisition process on a graph on a time axis on the display part (20).

## Description

### BACKGROUND ART

### 1. Technical Field

The present disclosure relates to an application program for a user, etc., provided specifically for use by users who are targets of myopia progression control. The application program, etc. are provided as effective countermeasures against increase in the myopia population from a preventive medicine perspective, which has never been seen before in ophthalmology, mainly targeting infants and children, against the background of a marked increase in the myopia population and a decreasing age of the myopia population in recent years.

### 2. Description of the Related Art

Myopia in the human eye requires a troublesome lifestyle requiring refraction correction means such as glasses and contact lenses. Additionally, it is also pointed out that the stronger the myopia, the more it hinders one's ability in sports and other activities, and that the risk of retinal detachment, glaucoma, and other lesions becomes greater due to aging. Particularly in recent years, the prevalence of myopia has increased significantly and myopia develops at younger ages, so that there is a growing social demand for prevention and effective control of myopia.

Besides, as myopia progresses, as mentioned above, the risks increase, the correction measures are required, and the physical and financial burden may increase. Thus, prevention and early treatment to control the progression of myopia in its early stages are important.

On the other hand, while preventive medicine is widespread in other medical fields, the actual situation is that few countermeasures have been taken in the field of ophthalmology from the perspective of preventive medicine. Therefore, it is difficult to prevent the progression of myopia or to undergo progression control treatment, despite the importance of early detection and progression control treatment of myopia. In particular, myopia often develops and progresses in the age group of infants and children, and in recent years the age group has become even younger. This makes it difficult for the targets to even recognize the onset and degree of progression of myopia by themselves, and poses a problem that it is difficult for the targets to recognize and complain of such progression, and countermeasures are likely to be late.

Additionally, with regard to myopia progression control treatment, in recent years, various procedures such as orthokeratology using contact lenses (see Japanese Unexamined Patent Publication No. JP-A-2017-138977, etc.) have been studied and some have been put into practical use. However, it is hard to say that such knowledge is widely spread among the general public.

Under these circumstances, the inventors conducted various discussions and studies targeting the users themselves who are the targets of prevention and progression control of myopia. These discussions and studies were conducted with the aim of raising awareness of the users from a preventive medicine perspective to prevent myopia, and disseminating knowledge about myopia progression control treatment, as well as realizing early treatment of myopia in its initial stage, thereby improving the effect of myopia progression control treatment, and the like.

From this perspective, the present inventors discussed, for example, providing an application for mobile terminals, etc. that would allow the users to grasp their own visual status by using vision (the index value based on Landolt rings) and corrective lens power (the diopter value), which are well known as indicators of myopia. However, it was difficult to measure vision and corrective lens power with sufficient accuracy and reliability for the users in the age group of infants and children. Besides, regarding vision and corrective lens power, it was statistically difficult to obtain information because of the large individual differences in future changes, making it difficult to providing significant information.

### SUMMARY

It is therefore one object of the present disclosure to provide an application program for a user, a myopia management device for a user, an ocular information processing method for a user, and an information processing system for a myopia management service which are able to provide a myopia management service that can disseminate prevention and progression control treatment of myopia from a new and unprecedented perspective, which are effective in recalling the preventive medicine perspective targeting ophthalmology by raising awareness of the users about myopia through direct use by the users themselves.

Hereinafter, preferred embodiments for grasping the present disclosure will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in the present disclosure, various other preferred embodiments can be realized without being limited to those described below.

A first preferred embodiment disclosure provides an application program for a user performing processes in a user terminal device including a display part, the user terminal device being used by the user who is a target of a myopia management service, the processes comprising: a measurement acquisition process acquiring at least one measurement of an ocular axial length of the user; and a measurement display process displaying the measurement acquired by the measurement acquisition process on a graph on a time axis on the display part.

With the application program according to the present preferred embodiment, the application program for the user who is the target of myopia prevention and progression control is provided. This makes it possible to raise awareness of the users about myopia prevention and progression control treatment by directly appealing to the user himself/herself. As a result, a new technical effect can be achieved by providing the myopia management service that can realize preventive medical treatment and early myopia progression control treatment targeting ophthalmology, which were not available in the past.

Specifically, with the application program according to the present preferred embodiment, the measurement of the user's ocular axial length is displayed on the graph on the time axis on the display part of the user terminal device used by the user.

Here, the ocular axial length, which until now has been used only professionally, is adopted as the ocular information displayed on the time axis. This is because myopia progression in infancy is often caused by the progression of axial myopia, and it is thought that with a hyperopic focal error that focuses on the outside behind the retina being a trigger, the retina extends so as to complement this hyperopic focal error to increase the ocular axial length, thereby causing myopia and its progression. Therefore, by measuring the ocular axial length, which was not very familiar to the users in the past, and displaying it on the graph on the display part of the user terminal device, it is possible to help the users understand the necessity for prevention and progression control treatment of myopia from a new perspective that did not exist in the past, and to provide the application program for the user which is able to provide the myopia management service that can disseminate preventive medical measures for myopia and myopia progression control treatment targeting ophthalmology.

That is, with the application program according to the present preferred embodiment, it is possible to raise awareness of the users from the perspective of preventive medical care to prevent myopia, and to disseminate knowledge about myopia progression control treatment, as well as to realize early treatment of myopia in its initial stage, thereby improving the effect of myopia progression control treatment. In particular, since user's ocular axial length can be used as the measurement, it is possible to measure and predict the existence or nonexistence and progression condition of myopia with sufficient accuracy and reliability, compared to the conventional case targeting the users in the age group of infants and children, in which the users answer their own visual status using Landolt rings.

The measurement acquisition process is not limited to the preferred embodiment which acquires the measurement by direct input operation from the user terminal device. For example, it would also be acceptable to provide the measurement measured by an ophthalmologist to the user terminal device via the Internet line or the like, or to specify an ophthalmologist's site from the user terminal device to pick up and acquire the measurement. In the future, when the ocular axial length measurement becomes more common, the user terminal device may directly measure the ocular axial length and acquire the measurement.

A second preferred embodiment provides the application program according to the first preferred embodiment, wherein the measurement acquisition process is performed a plurality of times with a time interval, and the at least one measurement of the ocular axial length comprises a plurality of measurements of the ocular axial length, and the measurement display process displays the plurality of measurements of the ocular axial length acquired by the measurement acquisition process performed the plurality of times on the graph on the display part.

By displaying the measurements of the ocular axial length acquired with a time interval on the graph on the display part, the user can easily check the effect of myopia prevention and the effect of myopia progression control treatment by himself/herself. This makes it possible to keep and improve the user's motivation for myopia prevention and myopia progression control. The display form of "the plurality of measurements of the ocular axial length" on the graph is not limited in any way. For example, it is also possible to display by plotting points, or to display in a line graph form.

A third preferred embodiment provides the application program according to the first or second preferred embodiment, wherein the processes further comprise: a prediction data acquisition process acquiring prediction data of a change mode of an ocular axial length in a future obtained based on the measurement acquired by the measurement acquisition process; and a prediction data display process displaying the prediction data acquired by the prediction data acquisition process on the graph on the time axis on the display part together with the measurement acquired by the measurement acquisition process.

For example, by initially inputting the measurement of the ocular axial length together with the age (e.g., 6 years old) and the diopter value (e.g., 2D), or alone, it is possible to alert the user to the fact that the user will become myopic in such a way (e.g., at 15 years old) if no action is taken, which is displayed on the graph based on the prediction data of the change mode of the ocular axial length, thereby raising awareness of myopia prevention. Furthermore, by displaying the prediction data of the change mode of the ocular axial length in the case of undergoing myopia progression control treatment together, it is also possible to motivate the user to actively participate in the myopia progression control treatment.

The "prediction data" can be obtained from a computed value, a published value in a research paper, as well as a statistical value, a selection or computing using statistical data, or by using an AI model, etc., for example. Besides, the prediction data may be acquired, for example, by inputting the data directly into the terminal device from the outside, by selecting appropriate data from a plurality of pieces of data (including statistical data, etc.) stored in a data server connected via an interface or by generating through computing using influencing factors (age, height, gender, current eye condition, etc.). Moreover, the prediction data may also be acquired from the information of a specific user by using an AI model that has been trained on a number of specific cases. Such preferred embodiments may also include acquiring the prediction data in cooperation with the external software connected via a network such as the Internet line.

A fourth preferred embodiment provides the application program according to the third preferred embodiment, wherein the prediction data acquired by the prediction data acquisition process includes prediction data of a change mode of an ocular axial length of an emmetropic eye.

Since the prediction data includes the prediction data of the change mode of the ocular axial length of the emmetropic eye, the change mode of the emmetropic eye (a nonmyopic eye) can be used as the ideal data. Moreover, by acquiring the prediction data of the change mode of the ocular axial length of the emmetropic eye based on the user's ocular axial length at the present time (at the time of measurement), it is possible to suppress an error caused by a large individual difference in the ocular axial length in particular. Then, for example, in combination with the second preferred embodiment, it is possible for the user, who has started management with an emmetropic eye, to objectively recognize whether or not his/her eye deviates from the change mode of the ocular axial length of the emmetropic eye over time. This is "significant and important from a preventive medicine perspective," and moreover, leads to the realization of "early treatment" in which the user undergoes early preventive medical care for myopia based on the knowledge of the myopia suspicion. That is, such an effect is not feasible with the conventionally known systems related to ophthalmology, and the new technical effect of realizing preventive medical treatment and early myopia progression control treatment targeting ophthalmology can be further advantageously achieved. The present preferred embodiment is able to effectively provide a new preventive medical system targeting ophthalmology that is of great social significance. Like the prediction data in the preceding first preferred embodiment, the "prediction data of the emmetropic eye" can be obtained from a computed value, a published value in a research paper, as well as a statistical value, a selection or computing using statistical data, or by using an AI model, etc., for example.

A fifth preferred embodiment provides the application program according to the third or fourth preferred embodiment, wherein the prediction data acquired by the prediction data acquisition process includes prediction data reflecting an effect of myopia progression control treatment.

Since the prediction data includes the prediction data reflecting the effect of the myopia progression control treatment, the user who encounters the prediction data reflecting the effect of the myopia progression control treatment displayed on the display part of the user terminal device can be advantageously motivated to continue the myopia progression control treatment. Like the prediction data in the preceding third preferred embodiment, the "prediction data reflecting the effect of the treatment" can be obtained from a computed value, a published value in a research paper, as well as a statistical value, a selection or computing using statistical data, or by using an AI model, etc., for example.

A sixth preferred embodiment provides the application program according to any one of the third through fifth preferred embodiments, wherein the processes further comprise: a measurement deviation acquisition process obtaining a deviation between the prediction data acquired by the prediction data acquisition process and the measurement acquired by the measurement acquisition process; and an alert process issuing an alert if a size of the deviation obtained by the measurement deviation acquisition process exceeds a predetermined allowable deviation range.

Since the alert is issued when the size of the deviation exceeds the predetermined allowable deviation range, the user can effectively recognize the case where there is a high necessity to undergo some preventive measure or progression control treatment. This makes it possible to further advantageously realize preventive medical treatment and early myopia progression control treatment targeting ophthalmology. Various types of alerts are adoptable for the display part, such as displaying a specific color, a blink, and a sound, for example. The alert can also be transmission by e-mail or other means to a specific external party. Besides, it would also be acceptable to employ a determination process determining the degree of deviation, and to vary the alerts depending on the degree of deviation. The "size of deviation" may be, for example, the deviation of the numerical value from the prediction data, as well as the deviation of the rate of change (the inclination of the graph representing time-dependent progress) from the prediction data.

A seventh preferred embodiment provides the application program according to the sixth preferred embodiment, wherein the alert of the alert process includes a consultation alert for myopia progression control treatment.

Since the alert includes the consultation alert for myopia progression control treatment, early myopia progression control treatment can be further advantageously realized. The "consultation alert for myopia progression control treatment" can further include an "ophthalmologist referral process introducing a nearby ophthalmologist who can provide myopia progression control treatment" and an "ophthalmologist appointment process arranging an appointment with the said ophthalmologist". The following describes the specific preferred embodiment of the ophthalmologist referral process and the ophthalmologist appointment process. For example, it would be acceptable to prepare an ophthalmologist database on a cloud computer or the like that stores information on a plurality of ophthalmologists, such as locations, business hours, specialties, past careers, and the like. By accessing and referring to the information on the said cloud computer from the user terminal device, the appropriate ophthalmologist's information may be acquired to be displayed on the display part of the user terminal device, or to be transmitted separately to the user's registered e-mail address, etc.

Besides, the user terminal device may also access an ophthalmologist terminal device used by the ophthalmologist through a communication line, such as the Internet line, to acquire information on the current status of crowdedness, availability of appointments, and the like of the ophthalmologist, for example, and to transmit the information to the member. It would also be acceptable to perform the appointment process through mutual communication, to transmit to the ophthalmologist information such as the history and symptoms of the member who wishes to see the ophthalmologist, and to make an appointment with the ophthalmologist.

An eighth preferred embodiment provides the application program according to any one of the third through seventh preferred embodiments, wherein the processes further comprise: a measurement deviation acquisition process obtaining a deviation between the prediction data acquired by the prediction data acquisition process and the measurement acquired by the measurement acquisition process; and an additional service process providing an additional service if a size of the deviation obtained by the measurement deviation acquisition process is within a predetermined allowable deviation range.

If the size of the deviation acquired by the measurement deviation acquisition process is within the predetermined allowable deviation range, it indicates that the user is currently in the state where myopia can be prevented or the effect of the myopia progression control treatment is exhibited. Therefore, the "additional service" includes, for example, displaying what pleases infants and children on the display part or the like, namely, a character display, a stamp with a predetermined design, an animation display, and awarding of points that can be exchanged for predetermined services, etc. This can improve the effect of the application program according to the present disclosure, which effectively prevents myopia and controls myopia progression, by increasing motivation of infants and children who are the main users of the program.

A ninth preferred embodiment provides the application program according to any one of the first through eighth preferred embodiments, wherein the processes further comprise an ocular refractive power value acquisition process acquiring an ocular refractive power value of the user.

If the refractive power value of the user's naked eye (including the so-called vision and the diopter value of the corrective lens, etc.) is also acquired, it is possible to correct or selectively adopt the future prediction data, including information such as, for example, "If the user is myopic to this degree at the current age, the myopia will worsen to that degree during the growth process," thereby making the display more accurate. Additionally, there is a merit that a general user is more familiar with the vision or the like than the ocular axial length, which makes it easier to assume (imagine) the situation. Suitably, the process displaying the acquired ocular refractive power value on the display part may be included.

A tenth preferred embodiment provides the application program according to any one of the third through eighth preferred embodiments, or the ninth preferred embodiment when dependent on the third preferred embodiment, wherein the processes further comprise: a future visual image acquisition process acquiring a future visual image based on the prediction data of the change mode of the ocular axial length in the future of the user acquired by the prediction data acquisition process; and a future visual image display process displaying the future visual image acquired by the future visual image acquisition process on the display part.

Even if an indication or alert is issued at the present time to tell that "myopia will progress" or "eyesight will deteriorate" in the future, it is difficult to imagine the situation as reality, and especially for infants and children, it is difficult to understand the situation in many cases. Therefore, it is effective from the perspective of early treatment and preventive medicine to let the users realize how their vision changes. This will increase the user's own motivation for myopia prevention and myopia progression control treatment, thereby improving the effect of the application for the user according to the present disclosure as well. Incidentally, the actual visual image can be acquired by using an external computing process, for example. Specifically, it is possible to make use of a "site to experience how myopia looks (Eye Sim)" or to display a still or moving image by performing a computing process with reference to such a site.

An eleventh preferred embodiment provides the application program according to any one of the third through eighth, and tenth preferred embodiments, or the ninth preferred embodiment when dependent on the third preferred embodiment, wherein in the prediction data display process, the prediction data is displayed as a band-shaped area extending on the time axis on the display part.

For example, in displaying the prediction data using statistical information, a form of displaying the predetermined percentile values as a band-shaped area may be adopted. This make it easy for the user to intuitively understand the data, including the deviation from the median, the average, or the like, thereby providing the application program as a more user-friendly application program. The prediction data may be displayed in a linear graph or in a form of points displayed at a predetermined time interval.

A twelfth preferred embodiment provides the application program according to any one of the first through eleventh preferred embodiments, wherein the measurement acquisition process is performed a plurality of times with a time interval, and the at least one measurement of the ocular axial length comprises a plurality of measurements of the ocular axial length, and the processes further comprise a change rate acquisition process obtaining a rate of change on the time axis for the plurality of measurements of the ocular axial length acquired by the measurement acquisition process performed the plurality of times.

Regarding the measurements of the ocular axial length acquired a plurality of times with a time interval, by acquiring the "rate of change," the change in the ocular axial length can be evaluated with the time axis, making it possible to more accurately grasp the tendency toward myopia. Besides, for example, in combination with the third preferred embodiment, it is possible to acquire the rate of change for the prediction data as well. This facilitates a more accurate grasp of the tendency toward myopia, the degree of effect of myopia progression control treatment, and the like by comparing the rates of change between the prediction data and the measurements.

A thirteenth preferred embodiment provides the application program according to any one of the first through twelfth preferred embodiments, wherein the processes further comprise: an imaging data acquisition process acquiring lens imaging data for a contact lens provided to the user for myopia progression control treatment; a lens condition determination process determining a condition of the contact lens from the lens imaging data acquired by the imaging data acquisition process; and a determination result output process outputting a determination result of the condition of the contact lens acquired by the lens condition determination process.

The contact lenses provided for myopia progression control treatment have a special shape, such as orthokeratology lenses, so as to be prone to contamination due to accumulation of protein and the like on the lens surface. Meanwhile, such contact lenses have special characteristics that due to the special usage method, such as wearing the lenses at bedtime, it is difficult to subjectively recognize the contamination based on changes in vision or the like. Therefore, the present preferred embodiment uses the imaging data (including both still and moving images) as objective information to objectively grasp the contamination, damage, and the like. This makes it possible to objectively grasp the lens condition, thereby maintaining and improving safety. Regarding the specific determination, for example, the degree of color and the size of the area (the number of pixels) of the imaged lens, the movement of the lens with eye movement and blinking during wearing in moving images, the tear film break-up time (BUT), and the like can be subject to evaluation. By imaging the lens with a specific reagent or the like attached, it is also possible to improve the determination accuracy of foreign matter adhesion. Besides, the determination result output process can display the result on the display part, as well as output it externally by linking with other applications (e.g., an e-mail, etc.), or the like.

A fourteenth preferred embodiment provides the application program according to the thirteenth preferred embodiment, wherein the processes further comprise a procedure instruction output process instructing the user about a necessary procedure if the determination result outputted by the determination result output process is defective.

If the determination result outputted by the determination result output process is defective (not normal), the procedure instruction output process instructing the user about specific procedures (e.g., cleaning, replacement, etc.) may be further included. This makes it possible for the user to receive appropriate myopia progression control treatment, thereby further improving myopia progression control effect. The procedure instruction outputted by the procedure instruction output process may include, for example, displaying a compatible cleaning solution, linking to a purchase process of the said cleaning solution, as well as guiding the lens replacement procedure, and, if necessary, an instruction to consult an ophthalmologist.

A fifteenth preferred embodiment provides the application program according to any one of the first through fourteenth preferred embodiments, wherein the processes further comprise: a treatment information acquisition process acquiring information of myopia progression control treatment prescribed to the user; and an information transmission process for an ophthalmologist transmitting the measurement acquired by the measurement acquisition process and the information of the myopia progression control treatment acquired by the treatment information acquisition process inclusively to an ophthalmologist terminal device used by the ophthalmologist.

According to the present preferred embodiment, continuous treatment can be efficiently expected even when the ophthalmologist changes due to the user's relocation or the like. Regarding the ophthalmologist terminal device, when the ophthalmologist is equipped with a separate database or uses the cloud, for example, the device includes the database or the cloud computer.

A sixteenth preferred embodiment provides the application program according to any one of the third through eighth, and tenth preferred embodiments, or any one of the ninth, and twelfth through fifteenth preferred embodiments when dependent on the third preferred embodiment, wherein the prediction data display process and the measurement display process further comprise a display mode change process changing a range of the time axis displayed on the display part to zoom in and out a display mode.

For example, when the myopia progression control treatment has been performed over a long period of time, for example, it is possible to meet the requests such as zooming in only a specific time axis range to view in detail, or zooming it out to check the change mode, as well as changing the display period including switching between short periods such as several months or six months, and long periods such as several years, thereby improving the convenience.

A seventeenth preferred embodiment provides the application program according to any one of the first through sixteenth preferred embodiments, wherein the processes further comprise: a life information acquisition process acquiring a life situation data of the user; and a life information display process displaying the life situation data acquired by the life information acquisition process together with time axis information on the display part.

Since the degree of myopia and its status are influenced by the life situation as well, functions of data accumulation and appropriate display on such a life situation may be imparted to the application program. Accordingly, for example, if myopia progression is faster in a certain time range or if myopia progression control treatment is more effective in a certain time range, it is possible to look back on the life situation in the said time range and compare it with that in other time ranges as necessary. This makes it easier for the user to make efforts toward a life situation that is expected to control myopia progression, and can provide the user with one indicator thereof. It can be assumed that the life situation data includes: for example, the type, presence or absence, and the time of club activities; the time of study (a cram school and home); the type, presence or absence, and the time of lessons; sleeping hours; sleeping and waking times; lens data in use and duration of wearing; the outdoor activity time; presence or absence and the time of use of electronic devices; presence or absence and duration of wearing of night treatment lens use; and the like, which are information that may be expected to influence changes in ocular axial length.

An eighteenth preferred embodiment provides the application program according to the seventeenth preferred embodiment, wherein the life situation data of the user acquired by the life information acquisition process includes an outdoor activity time, and the processes further comprise an outdoor activity alert process issuing an outdoor activity alert that prompts the user to do an outdoor activity for a certain amount of time or more if the outdoor activity time is below a predetermined value.

By acquiring the outdoor activity time that has a significant influence on myopia in infants and children and issuing an alert prompting the user to take outdoor activity time that is effective in myopia progression control, the myopia prevention effect and the myopia progression control effect can be further improved.

A nineteenth preferred embodiment provides the application program according to any one of the first through eighteenth preferred embodiments, wherein the processes further comprise: a member information acquisition process acquiring member registration information of the user; and a member information display process displaying the member registration information of the user acquired by the member information acquisition process on the display part.

With membership as a requirement, the application program can be made available by appropriately selecting personally identifiable information such as the user's address, name, age, and prescription information acquired by the member information acquisition process. In the case where payment of a membership fee is a requirement for membership registration, an application usage permission process can be further included. In this process, a confirmation process confirming payment of the membership fee is also employed, for example, and if fulfillment of the membership requirement including the payment of the membership fee is confirmed by the confirmation process, the functions of the application program becomes available.

A twentieth preferred embodiment provides the application program according to the second preferred embodiment, or any one of the third through nineteenth preferred embodiments when dependent on the second preferred embodiment, wherein the plurality of measurements of the ocular axial length acquired by the measurement acquisition process performed the plurality of times include the measurement during treatment of myopia progression control treatment and the measurement during a stop of the myopia progression control treatment, and the measurement of the ocular axial length acquired during the treatment of the myopia progression control treatment and the measurement of the ocular axial length acquired during the stop of the myopia progression control treatment that are displayed on the display part by the measurement display process are distinguishable on the graph.

According to the present preferred embodiment, the measurements of the ocular axial length acquired during the treatment of the myopia progression control treatment and the measurements of the ocular axial length acquired during the stop of the myopia progression control treatment are distinguishable on the graph displayed on the display part. Thus, for example, the effect of the myopia progression control treatment can easily be visually grasped. For example, if the effectiveness of the myopia progression control treatment is sufficiently confirmed (i.e., the extension of the ocular axial length is sufficiently controlled), the treatment can be stopped. Meanwhile, if the extension of the ocular axial length has increased to some extent after a predetermined period of time has passed since the treatment is stopped, the myopia progression control treatment can be restarted, or the like. Such a preferred embodiment may be one of the factors to determine whether or not the myopia progression control treatment is necessary.

A twenty-first preferred embodiment provides an information processing system comprising: the application program according to any one of the first through twentieth preferred embodiments; and an information processing device that is communicable with the user terminal device used by the user, wherein the information processing device includes storage means for storing statistical data of the ocular axial length.

By including the application program according to any one of the first through twentieth preferred embodiments in the information processing system, it is possible to provide the information processing system for the myopia management service that can achieve the same working effects as those of using the application program according to any one of the first through twentieth preferred embodiments.

A twenty-second preferred embodiment provides the information processing system according to the twenty-first preferred embodiment, further comprising: computing means for obtaining the prediction data according to the third preferred embodiment based on the measurement of the ocular axial length of the user by using the statistical data of the ocular axial length stored in the storage means; and transmission means for transmitting the prediction data obtained by the computing means to the user terminal device.

With the information processing system according to the present preferred embodiment, it is possible to obtain the prediction data according to the third preferred embodiment, and to provide the information processing system that can achieve the same working effects as those of using the application program utilizing the said prediction data.

A twenty-third preferred embodiment of the present disclosure provides a myopia management device for a user used by the user who is a target of a myopia management service, the device comprising a display part displaying a measurement of an ocular axial length specific to the user on a time axis, wherein the measurement of the ocular axial length is displayed on the display part.

The myopia management device according to the present preferred embodiment can have the same benefit of the above-mentioned working effects as those brought about by the application program according to the first preferred embodiment. This makes it possible to help the users understand the necessity for prevention and progression control treatment of myopia, and to provide the myopia management device which is able to provide the myopia management service that can disseminate preventive medical measures for myopia and myopia progression control treatment targeting ophthalmology.

A twenty-four preferred embodiment of the present disclosure provides the myopia management device according to the twenty-third preferred embodiment, wherein prediction data of a change mode of the ocular axial length in a future obtained based on the measurement of the ocular axial length of the user is displayed on the display part together with the measurement.

The myopia management device according to the present preferred embodiment can use the prediction data according to the third preferred embodiment, for example. This makes it possible to provide the myopia management device that can have the same benefit of the above-mentioned working effects as those brought about by the application program using the said prediction data.

A twenty-fifth preferred embodiment of the present disclosure provides an ocular information processing method for a user performed by a user terminal device used by the user who is a target of a myopia management service, the method comprising: acquiring and storing a measurement of an ocular axial length of the user; and displaying the stored measurement of the ocular axial length on a graph on a time axis on a display part.

The ocular information processing method according to the present preferred embodiment can have the same benefit of the above-mentioned working effects as those brought about by the application program according to the first preferred embodiment. This makes it possible to help the users understand the necessity for prevention and progression control treatment of myopia, and to provide the ocular information processing method which is able to provide the myopia management service that can disseminate preventive medical measures for myopia and myopia progression control treatment targeting ophthalmology.

A twenty-sixth preferred embodiment of the present disclosure provides the ocular information processing method according to the twenty-fifth preferred embodiment, further comprising: acquiring and storing prediction data of a change mode of the ocular axial length in a future of the user, the prediction data being obtained based on the measurement of the ocular axial length; and displaying the stored measurement of the ocular axial length and the stored prediction data of the ocular axial length together on the graph on the display part.

The ocular information processing method according to the present preferred embodiment can use the prediction data according to the third preferred embodiment, for example. This makes it possible to provide the ocular information processing method that can have the same benefit of the above-mentioned working effects as those brought about by the application program using the said prediction data.

According to the present disclosure, it is possible to provide an application program for a user, a myopia management device for a user, an ocular information processing method for a user, and an information processing system which are able to provide a myopia management service that can disseminate prevention and progression control treatment of myopia from a new and unprecedented perspective, which are effective in recalling the preventive medicine perspective targeting ophthalmology by raising awareness of the users about myopia through direct use by the users themselves.

### BRIEF DESCRIPTION OF THE DRAWINGS

The foregoing and/or other objects, features and advantages of the disclosure will become more apparent from the following description of a practical embodiment with reference to the accompanying drawings in which like reference numerals designate like elements and wherein:
FIG. 1 shows an overall structure of an information processing system for a myopia management service according to a first practical embodiment of the present disclosure;
FIG. 2 is a flowchart showing a specific example of an action to make an application program available in a case where the myopia management service is provided with membership as a requirement;
FIG. 3 is a flowchart showing a specific example of an action of acquiring prediction data of a change mode of a user's ocular axial length in the future using an application program for a user and displaying the data on a display part of a user terminal device;
FIG. 4 shows a specific example of data structure stored in acquired measurement information in a storage part of an information processing device;
FIG. 5 shows details of an action of the information processing system in a prediction data acquisition process;
FIG. 6 shows a specific example displaying the prediction data acquired by the prediction data acquisition process together with a user's ocular axial length acquired by a measurement acquisition process on the display part of the user terminal device;
FIG. 7 is a flowchart showing a specific example of an action of the information processing system in a case where user's life situation data acquired by a user life information acquisition process includes an outdoor activity time;
FIG. 8 shows another specific example displaying the prediction data acquired by the prediction data acquisition process together with the user's ocular axial length acquired by the measurement acquisition process on the display part of the user terminal device;
FIG. 9 is a flowchart showing a specific example of an action of the information processing system in a case where the measurement acquisition process is performed a plurality of times with a time interval;
FIG. 10 shows yet another specific example displaying the prediction data acquired by the prediction data acquisition process together with the user's ocular axial length acquired by the measurement acquisition process on the display part of the user terminal device;
FIG.11 is a flowchart showing another specific example of an action of the information processing system in a case where the measurement acquisition process is performed a plurality of times with a time interval;
FIG. 12 shows still yet another specific example displaying the prediction data acquired by the prediction data acquisition process together with the user's ocular axial length acquired by the measurement acquisition process on the display part of the user terminal device;
FIG. 13 is a flowchart showing yet another specific example of an action of the information processing system in a case where contact lenses for myopia progression control treatment are used; and
FIG. 14 shows a further specific example displaying the user's ocular axial length acquired by the measurement acquisition process on the display part of the user terminal device.

### DETAILED DESCRIPTION

A practical embodiment of the present disclosure, namely, an application program for a user, an information processing system for a myopia management service, a myopia management device for a user, and an ocular information processing method for a user, which are able to provide a myopia management service that can disseminate prevention and progression control treatment of myopia from a new and unprecedented perspective, will be described below in reference to the drawings.

### System configuration

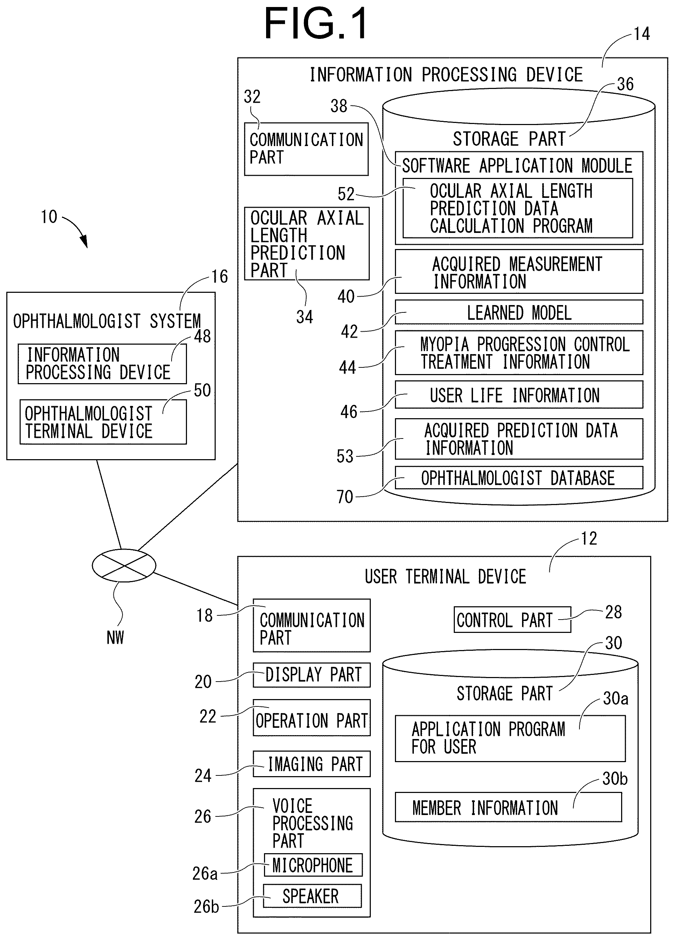

FIG. 1 illustrates the overall configuration of an information processing system 10 for myopia management service that is able to provide a myopia management service based on a novel perspective. Here, the myopia management service can be operated by an organization that aims to promote myopia prevention and myopia progression control treatment, and can be managed and operated by, for example, a contact lens manufacturer that provides an ocular device for myopia progression control, an ophthalmologist, and the like. The information processing system 10 includes a user terminal device 12 and an information processing device 14. The user terminal device 12 and the information processing device 14 communicate via a network NW. The network NW includes, for example, a part or all of a WAN (Wide Area Network), a LAN (Local Area Network), the Internet, a provider device, a wireless base station, a leased line, and the like. In FIG. 1, the user terminal device 12 and the information processing device 14 are shown independently of each other. However, the user terminal device 12 may have a part of the functions of the information processing device 14 described below, and vice versa. Besides, a part of the functions of the user terminal device 12 and the information processing device 14 may be performed in cooperation with other external information processing devices communicating via the network NW or with external software interfaced by an API (application programming interface). For example, the information processing system 10 can further communicate with an ophthalmologist system 16 via the network NW.

The user terminal device 12 is a mobile phone such as a smartphone, a tablet computer, or the like, used by users including infants and children, who are the targets of the myopia management service. The user terminal device 12 may be a desktop PC, a laptop PC, or a wearable terminal such as an HMD (Head Mount Display) and a wristwatch-type terminal. Although only one user terminal device 12 is shown in FIG. 1 for convenience, the user terminal device 12 is owned by each user of the myopia management service provided by the information processing system 10, and a number of user terminal devices 12 may be communicably connected to the information processing device 14 and the ophthalmologist system 16 via the network NW.

The user terminal device 12 includes a communication part 18, a display part 20, an operation part 22, an imaging part 24, a voice processing part 26, a control part 28, and a storage part 30.

The communication part 18 is a wireless communication module that performs wireless communication with a wireless base station connected to the network NW. The display part 20 is a display device such as a liquid crystal display device. The operation part 22 is a device that accepts operation instructions from the user, and can comprise, for example, a pointing device such as a touch panel, a touch pad, and a mouse, and a keyboard, or the like. The display part 20 and the operation part 22 may be configured as a touch panel display.

The imaging part 24 images an imaging picture of a subject by visible light and acquires it as image data, and can comprise a camera or the like. The voice processing part 26 performs conversion processing of voice signals between digital and analog, for example, and converts the voice signals given by a microphone 26a into digital signals to give the converted signals to the control part. The voice processing part 26 also gives the voice signals to a speaker 26b, and the speaker 26b converts the voice signals to voice and outputs it to the outside of the user terminal device 12.

The control part 28 is realized by a processor, such as a CPU (Central Processing Unit), executing an application program 30a for a user stored in the storage part 30. The control part 28 may be realized by hardware such as an LSI, an ASIC, and an FPGA having the same function as the processor executing the program, or by software and hardware working together.

The storage part 30 is realized by, for example, a RAM (Random Access Memory), a ROM (Read Only Memory), an HDD (Hard Disk Drive), a flash memory, or a hybrid type storage device that combines several of these. The storage part 30 stores the application program 30a and member information 30b. Details of the application program 30a and the member information 30b will be described later.

Next, the information processing device 14 will be described. The information processing device 14 is a computer that provides the user terminal device 12 with prediction data of the ocular axial length in the future and the like. The information processing device 14 includes a communication part 32, an ocular axial length prediction part 34, and a storage part 36. In FIG. 1, the information processing device 14 is illustrated as a single device for convenience. However, the information processing device 14 does not have to be a physically single device, but each function may be built using an external application on a server or the cloud in a suitable location that is communicably connected via the network NW.

The communication part 32 is a communication interface for connecting to the network NW. The communication part 32 may comprise, for example, a network interface card.

The ocular axial length prediction part 34 is realized, for example, by a hardware processor such as a CPU executing a program stored in the storage part 36 (such as an ocular axial length prediction data calculation program 52 described later). The program may be stored in advance in a storage device such as an HDD and a flash memory (a storage device equipped with a non-transitory storage medium). Alternatively, the program may be stored in a removable storage medium such as a DVD and a CD-ROM (a non-transitory storage medium), so as to be installed in the storage device when the storage medium is attached to a drive device.

The storage part 36 is an HDD, a flash memory, a RAM, or the like. The storage part 36 may be a NAS (Network Attached Storage) device accessible by the information processing device 14 via the network NW. The storage part 36 stores information such as a software application module 38, acquired measurement information 40, a learned model 42, myopia progression control treatment information 44, and user life information 46. The software application module 38 can include the ocular axial length prediction data calculation program 52 described later, and the application program 30a that can be downloaded to the user terminal device 12 via a communication line, as well as the API (application programming interface), and the like. Therefore, the ocular axial length prediction part 34 may calculate ocular axial length prediction data in cooperation with an external software interfaced by the API (application programming interface). The storage part 36 can be an example of storage means for storing statistical data of the ocular axial length, for example, in the information processing device 14. Furthermore, the information processing device 14 may include computing means for obtaining prediction data based on the user's ocular axial length by using the statistical data of the ocular axial length stored in the said storage means. The prediction data obtained by this computing means can be transmitted to the user terminal device 12 via transmission means comprising, for example, the communication part 32 and the network NW described above. Details of these program and information stored in the storage part 36 will be described later.

Next, the ophthalmologist system 16 will be described. The ophthalmologist system 16 is a system constructed within an ophthalmological clinic and includes, for example, an information processing device 48 and an ophthalmologist terminal device 50, which are connected to each other such that data communication is possible via a network within the ophthalmological clinic. The ophthalmologist system 16 is accessible via the network NW. Although only one ophthalmologist system 16 is shown in FIG. 1 for convenience, two or more ophthalmologist systems 16 may be interfaced via the network NW.

The storage part of the information processing device 48 (not shown) stores, for example, user information as well as information regarding the user's myopia progression control treatment, information regarding the time-dependent measurement results of the user's ocular axial length and ocular refractive power, and the like. Like the user terminal device 12, the ophthalmologist terminal device 50 comprises a communication part, a display part, an operation part, a control part, and a storage part, which are not shown in the drawings, and may comprise a desktop PC, a laptop PC, a mobile phone such as a smartphone, a tablet computer, or the like.

### Acquisition of member registration information

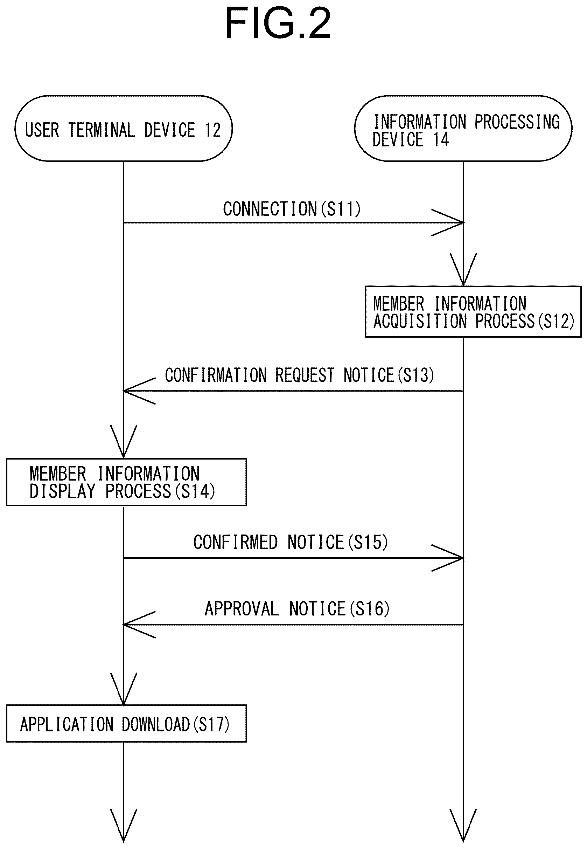

FIG. 2 is a flowchart showing an example of an action of making the application program available on the condition of acquisition of member registration information in the case where the myopia management service is provided with membership as a requirement. First, at S11, a user (a target in the age group of infants and children or a guardian thereof) who wishes to use the myopia management service accesses the application download site of the information processing device 14 via the network NW by operating the user terminal device 12. At S12, the user operates the user terminal device 12 to input and transmit the required member information 30b regarding the user, such that the member information acquisition process is performed in the information processing device 14. The member information 30b can include a user ID, the user's name, residence, gender, date of birth, ophthalmologist information, insurance card number, social ID number, and the like. Next, at S13, the inputted member information with a confirmation request notice is transmitted from the information processing device 14 to the user terminal device 12. At the subsequent S14, the registered member information is displayed on the display part 20 of the user terminal device 12, and the member information display process is performed. Afterwards, at S15, when the user terminal device 12 transmits a confirmed notice (including correction information if any), then at the following S16, an approval notice is transmitted from the information processing device 14. Accordingly, at S17, the application program 30a can be downloaded to the user terminal device 12, and stored in the storage part 30 together with the displayed member information 30b. In the case where payment of the membership fee is a requirement for membership registration, an application usage permission process requiring payment of the membership fee can be further included at the time of initial membership registration, subsequent login, and the like. Besides, the application program 30a may be distributed to the user terminal device 12 through a general digital content distribution service, for example. The application program 30a may be made available by the user inputting the member information 30b after downloading the application program 30a.

### Acquisition and display of prediction data of ocular axial length

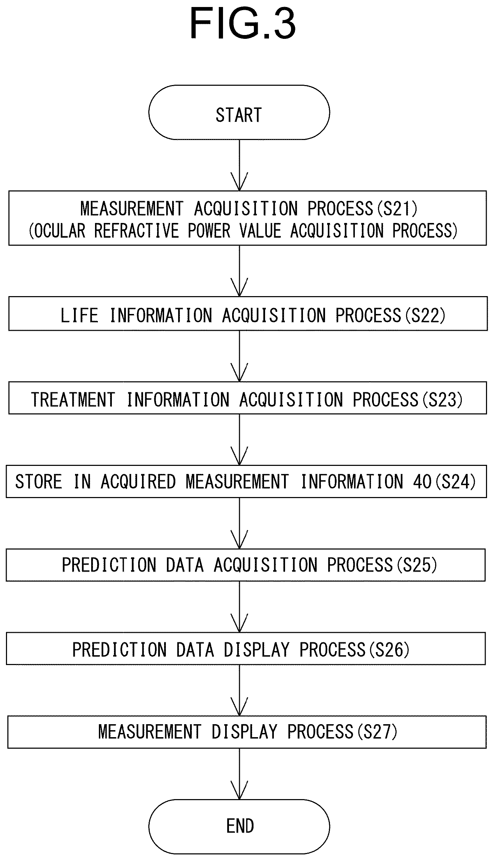

FIG. 3 is a specific example of a flowchart showing an example of an action of acquiring prediction data of a change mode of the user's ocular axial length in the future using the application program 30a and displaying the data on the display part 20 of the user terminal device 12. First, at S21, a measurement acquisition process is performed to acquire a measurement of the user's ocular axial length by operating the user terminal device 12. The acquisition of the measurement of the ocular axial length may be performed by, for example, a method of the user inputting the measurement of the ocular axial length measured by an ophthalmologist, etc., or a method of acquiring the measurement stored in the information processing device 48 of the ophthalmologist system 16 connected via the network NW. Alternatively, the measurement of the user's ocular axial length may be acquired from an ocular axial length measuring device (not shown) connected to the network NW. Besides, an ocular refractive power value acquisition process acquiring the user's ocular refractive power value in addition to the measurement of the user's ocular axial length may be simultaneously performed at S21. This is because the user's ocular refractive power value (including the naked vision, the diopter value of the corrective lens, etc.) is often acquired concomitantly during measurement of the user's ocular axial length. The user's ocular refractive power value can also be acquired in the same manner as the measurement of the ocular axial length, using the various acquisition methods exemplified above.

Preferably, at the subsequent S22, a life information acquisition process (S22) is performed, in which user's life situation data is acquired through input by the user operating the user terminal device 12. The life situation data may include: for example, the type, presence or absence, and the time of club activities; the time of study (a cram school and home); the type, presence or absence, and the time of lessons; sleeping hours; sleeping and waking times; lens data in use and duration of wearing; the outdoor activity time; presence or absence and the time of use of electronic devices; presence or absence and duration of wearing of night treatment lens; and the like, which are information that may be expected to influence changes in ocular axial length. For example, the time of use of the user terminal device 12 can be adopted as the time of use of the electronic devices. In the case where the situation of use and the time of use of the user terminal device 12 are recorded in the user terminal device 12, the mode of acquisition of the time of use of the electronic devices as the life situation data is not limited to input by the user, but may include acquisition from the user terminal device 12 automatically.

If the user is undergoing myopia progression control treatment, a treatment information acquisition process is performed at the subsequent S23. The acquisition of the treatment information may be performed by a method of the user operating the user terminal device 12 and inputting the user's treatment information data, or a method of acquiring the treatment information stored in the information processing device 48 of the ophthalmologist system 16 connected via the network NW. The treatment information may include information of treatment methods such as administration of low-concentration atropine eye drops, orthokeratology, and red light treatment, and prescription medicines and the like.

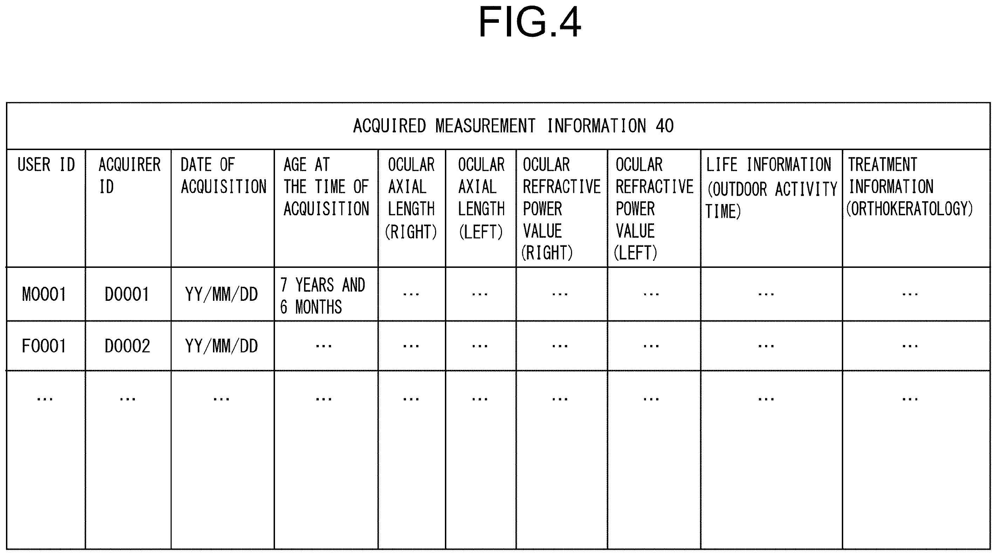

The measurement of the ocular axial length and the ocular refractive power value of the user acquired at S21, the life situation acquired at S22, and the treatment information acquired at S23 are transmitted to the information processing device 14 at S24 and stored in the acquired measurement information 40 in the storage part 36, together with the user ID and the date of acquisition. This makes it possible to accumulate measurements in the case where the measurement acquisition process (S21) is performed a plurality of times with a time interval of, for example, every 3 to 6 months. Moreover, the user's life situation and treatment information acquired at S22-23 are also stored in the acquired measurement information 40 in the storage part 36, together with the user ID and the date of acquisition. As a result, changes over time in user's ocular axial length and ocular refractive power value can be confirmed, which is able to contribute to confirming the effects of myopia preventive medical care and myopia progression control treatment and to improving the accuracy of prediction of future myopia status. Additionally, based on the acquired life situation, it is possible to look back on the life situation such as the outdoor activity time that may influence changes in ocular axial length, and this can provide a trigger or increase motivation to make efforts to improve the life situation that is expected to control myopia progression. FIG. 4 shows an example of the data structure stored in the acquired measurement information 40 in the storage part 36.

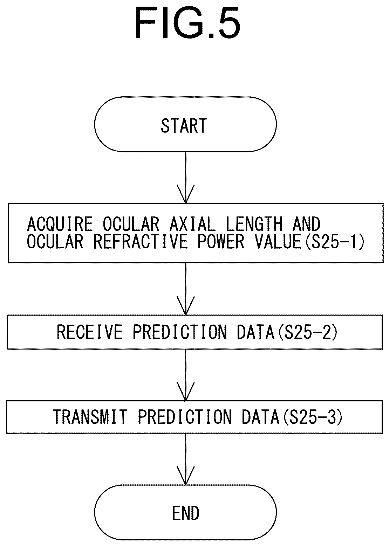

At the subsequent S25, the ocular axial length prediction part 34 of the information processing device 14 acquires the ocular axial length prediction data according to the ocular axial length prediction data calculation program 52, which is the program stored in the software application module 38 of the storage part 36, and a prediction data acquisition process (S25) is performed. FIG. 5 shows details of an action of the information processing system 10 in the prediction data acquisition process (S25). In S25-1, the ocular axial length prediction part 34 acquires the acquired ocular axial length and ocular refractive power value of the user acquired within a predetermined period of time from the acquired measurement information 40, together with data such as the age and gender at the time of acquisition, the life situation, the treatment information of the myopia progression control treatment, and the like. At the following S25-2, based on the data acquired at S25-1, prediction data including, for example, the change mode of the ocular axial length (such as extension) in the future are received from the learned model 42 in the storage part 36. The learned model 42 is trained to output prediction data indicating the predicted change mode of the ocular axial length over time, for example, one to several years into the future, in response to the input of data acquired at S25-1, such as the ocular axial length, the ocular refractive power value, the age and gender at the time of acquisition, the life situation, the treatment information of the myopia progression control treatment of the user, and the like, for example.

The learned model 42 is generated by having a model learning program perform machine learning, and is, for example, a parameterized composite function comprising a plurality of functions. The parameterized composite function is defined by a combination of multiple adjustable functions and parameters. Any parameterized composite function may be acceptable as long as it can derive the prediction data of the change mode of the ocular axial length corresponding to the input information described above. The multilayer network used to generate the learned model 42 can be, for example, a Deep Neural Network (DNN), which is a multilayer neural network subject to Deep Learning. For example, a Convolution Neural Network (CNN) targeting images may be used as the DNN.

Receiving the prediction data at S25-2 is not limited to using the learned model 42 exemplified above. For example, the "prediction data" may be a computed value derived from a formula using the acquired information as parameters, or may be a published value in a research paper or the like derived from the acquired measurement. Furthermore, the "prediction data" may be a statistical value related to the extension of the ocular axial length, or may be a selection or computing using statistical data. Specifically, the prediction data may be acquired using existing software for analyzing an ocular axial length in children, such as "Axial Manager (registered trademark)" made by TOMEY CORPORATION, which is capable of predicting the future myopia progression by analyzing the extension of the ocular axial length.

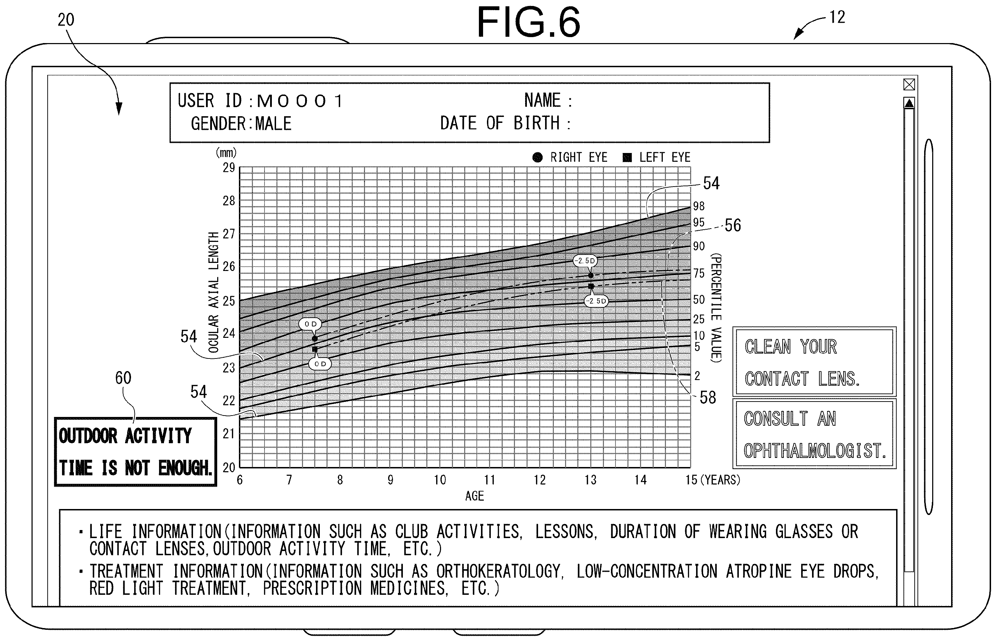

At the subsequent S25-3, the ocular axial length prediction part 34 stores the prediction data of the change mode of the ocular axial length in the future received at S25-2 together with the user ID in the acquired prediction data information 53 in the storage part 36, and transmits them to the user terminal device 12. This completes the prediction data acquisition process (S25). Returning to FIG. 3, at the following S26, on the display part 20 of the user terminal device 12, a prediction data display process (S26) is performed to display the prediction data acquired by the prediction data acquisition process (S25) on a graph on a time axis. FIG. 6 shows an example of the prediction data displayed on the graph on the time axis. The horizontal axis represents the age axis, showing the age range from 6 to 15 years. The vertical axis represents the ocular axial length. On the graph, an ocular axial length percentile curve 54 of an elementary or junior high school boy is shown as a colored area. The mode of coloring is not limited, but in FIG. 6, for example, the higher up the graph, the darker the coloring is, indicating that the degree of myopia is increasing. The ocular axial length percentile curve 54 includes curves showing percentile values of 2, 5, 10, 25, 75, 90, 95, and 98. At least the 10th, 25th, 75th, and 90th percentile curves and the band-shaped area between them can be grasped as the prediction data of the change mode of the ocular axial length of an emmetropic eye. This makes it possible for the user, who has started management with an emmetropic eye, to objectively recognize whether or not his/her eye deviates from the change mode of the ocular axial length of the emmetropic eye over time, thereby disseminating preventive medicine for myopia in an easy-to-understand way.

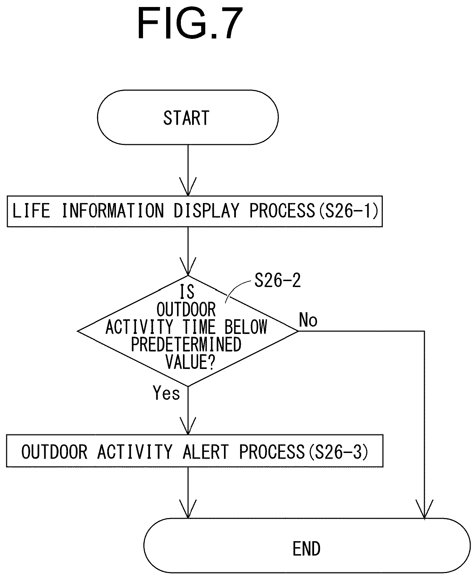

At the subsequent S27, on the display part 20 of the user terminal device 12, a measurement display process (S27) is performed to display the measurement acquired by the measurement acquisition process (S21) together with the graph displayed at the prediction data acquisition process (S26). In the example shown in FIG. 6, the measurements of the user, a 7-year-and-6-month-old boy, when his ocular axial length is measured for the first time are displayed on the graph together with the prediction data. On the graph, the black circle plots the measurement of the ocular axial length of the right eye, and the black square plots the measurement of the ocular axial length of the left eye. Near the black circle and the black square, the ocular refractive power value (0D) of the right eye and the ocular refractive power value (0D) of the left eye are displayed in a balloon shape. The curve indicated by the dot-and-dash line extending from the black circle to the right side is a right eye prediction curve 56, which shows the change mode of the ocular axial length of the right eye in the future. The curve indicated by the chain double-dashed line extending from the black square to the right side is a left eye prediction curve 58, which shows the change mode of the ocular axial length of the left eye in the future. At 7 years and 6 months old, when the measurements were taken, the left and right ocular axial lengths were both near the 50th percentile, and it can be easily confirmed from the ocular refractive power values (0D) shown nearby that he has emmetropic eyes. However, at the age of 13, both the right eye prediction curve 56 and the left eye prediction curve 58 are near the 75th percentile, and the predicted ocular refractive power value is (-2.5D). This is influenced by the fact that the outdoor activity time was below the predetermined value (e.g., 1 hour/day), which was turned out from the user's life situation data obtained at S25-1, and an outdoor activity alert 60 is blinking on the graph in FIG. 6. That is, the prediction data display process (S26) shown in FIG. 3 may include the steps shown in FIG. 7. Because the patent drawings are in black and white, the measurements of the ocular axial lengths in the left and right eyes are indicated by the black circle and the black square in FIG. 6, and the right eye prediction curve 56 and the left eye prediction curve 58 are indicated by the dot-and-dash line and the chain double-dashed line. However, the display part 20 of the actual user terminal device 12 may use multiple colors, and for example, the measurements of the ocular axial lengths in the left and right eyes may be indicated by a red circle and a blue circle respectively, and the right eye prediction curve 56 and the left eye prediction curve 58 may be indicated by red and blue solid lines respectively.

As shown in FIG. 7, if the user's life situation data acquired by the user life information acquisition process (S22) includes the outdoor activity time, at S26-1, a life information display process is performed, in which the outdoor activity time is displayed together on the time axis information (the graph in FIG. 6) displayed on the display part 20. Then, at S26-2, it is checked whether the outdoor activity time is below the predetermined value (e.g., 1 hour/day). If the result is YES at S26-2, at S26-3, an outdoor activity alert process is performed, in which the outdoor activity alert 60 that prompts the user to do an outdoor activity for a certain amount of time or more is issued on the graph in FIG. 6. The outdoor activity alert 60 includes, for example, blinking the display frame of the outdoor activity time displayed on the graph in FIG.6, and displaying a message prompting an outdoor activity.

As shown in the example in FIG. 6, there may be cases where, even if the user has an emmetropic eye at the time of measurement of the ocular axial length, the user has a risk of myopia progression being accelerated due to lifestyle habits acquired by the life information acquisition process (S22). In such cases, it is possible to show the user the lifestyle habits that need to be improved with an alert notifying that the ocular axial length is extending and his eye is gradually moving away from the emmetropic eye. The user can easily confirm the difference between the change mode of the emmetropic eye displayed on the graph in FIG. 6 and the change mode of the prediction data, while being motivated to improve his life situation, such as increasing the outdoor activity time, so as not to move away from the change mode of the ocular axial length of the emmetropic eye. This makes it possible to prompt voluntary action by the user to achieve preventive medical care for myopia.

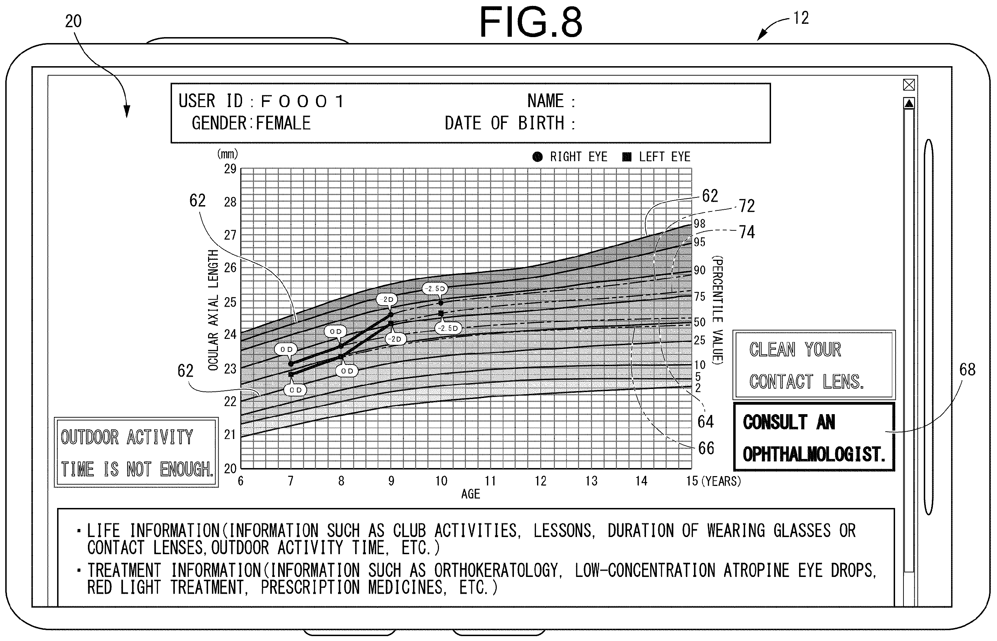

FIG. 8 shows another example of the prediction data displayed on the graph on the time axis. The horizontal axis represents the age axis, showing the age range from 6 to 15 years. The vertical axis represents the ocular axial length. On the graph, an ocular axial length percentile curve 62 of an elementary or junior high school girl is shown as a colored area. The ocular axial length percentile curve 62 includes curves showing percentile values of 2, 5, 10, 25, 75, 90, 95, and 98. At least the 10th, 25th, 75th, and 90th percentile curves and the band-shaped area between them can be grasped as the prediction data of the change mode of the ocular axial length of an emmetropic eye. Like the graphical display in FIG. 6, it is possible to disseminate preventive medicine for myopia in an easy-to-understand way.

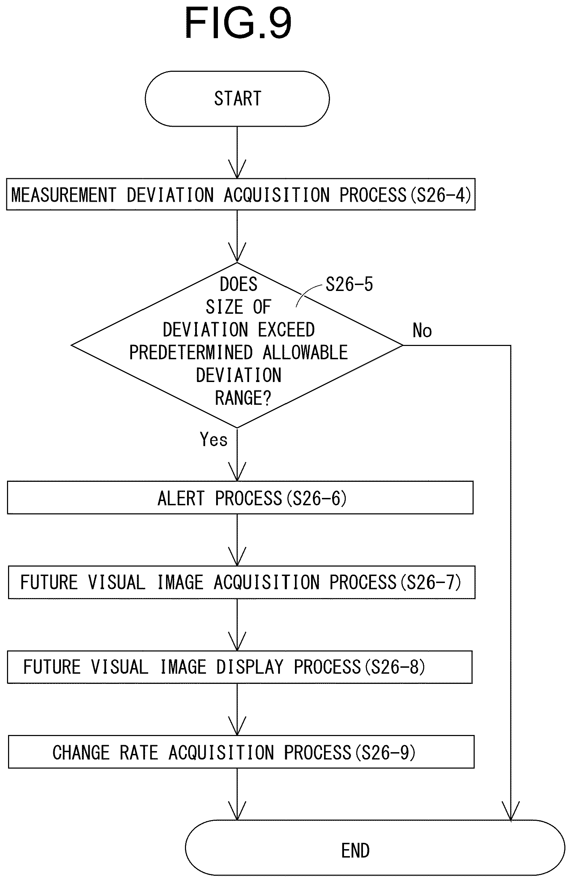

In the example shown in FIG. 8, the measurement acquisition process (S21) is performed three times with a time interval, including the times when the user, a 9-year-old girl, was 7 and 8 years old, and the measurements of the ocular axial length taken at the past three times are displayed on the graph together with the prediction data. At the ages of 7 and 8, the left and right ocular axial lengths were both near the 50th percentile, and it can be easily confirmed from the ocular refractive power values (0D) shown nearby that she had emmetropic eyes. However, at the age of 9, both the left and right ocular axial lengths are in the 75th to 90th percentile, which are significantly deviated from a right eye prediction curve 64 and a left eye prediction curve 66 at the age of 8, indicated by the dot-and-dash line and the chain double-dashed line, respectively. In the example shown in FIG. 8, the prediction data display process (S26) shown in FIG. 3 may include the steps shown in FIG. 9.

As shown in FIG. 9, in the case where the measurement acquisition process (S21) is performed a plurality of times with a time interval, at S26-4, a measurement deviation acquisition process is performed to obtain the deviation between the predicted ocular axial length at the age of 9 in the prediction data (the right eye prediction curve 64 / the left eye prediction curve 66), which was acquired by the prediction data acquisition process previously (at age 8), and the measurement of the ocular axial length, which is acquired by the measurement acquisition process (S21) this time (at age 9). Then, at S26-5, it is checked whether or not the size of the deviation obtained by the measurement deviation acquisition process (S26-4) exceeds the predetermined allowable deviation range. The "size of deviation" may be the amount of increase in the measurement of the ocular axial length at the age of 9 from the ocular axial length at the age of 9 in the prediction data, or may be the rate of change from the prediction data. For example, in the example in FIG. 8, the measurement of the ocular axial length at the age of 9 has increased by more than 0.6 mm from the prediction data at the age of 9, so that the determination at S26-5 is YES. If the determination at S26-5 is YES (namely, the size of deviation exceeds the predetermined allowable deviation range), an alert process is performed to issue an alert at S26-6. For example, the alert process may blink a consultation alert 68 for myopia progression control treatment on the graph in FIG. 8, for consulting an ophthalmologist and checking whether or not the myopia progression control treatment is required, and the method, the degree, and the like thereof, for example. Preferably, the consultation alert 68 may further include an "ophthalmologist referral process introducing a nearby ophthalmologist who can provide myopia progression control treatment" and an "ophthalmologist appointment process arranging an appointment with the said ophthalmologist". In order to receive the consultation alert 68 and consult an ophthalmologist who can provide myopia progression control treatment, the storage part 30 of the information processing device 14 may further store an ophthalmologist database 70 that stores locations, business hours, specialties, past careers, and the like for a plurality of ophthalmologists. The consultation alert 68 is not limited to blinking on the graph in FIG. 8, but may also be transmission of information to a registered e-mail address, etc. stored in the member information 30b of the user.

Furthermore, on the graph displayed on FIG. 8, the measurement acquired by the measurement acquisition process (S21) at this time (at age 9) is displayed by the measurement display process (S27). On the graph, the black circle plots the measurement of the ocular axial length of the right eye, and the black square plots the measurement of the ocular axial length of the left eye. Near the black circle and the black square, the ocular refractive power values (-2D) of the right and left eyes are displayed in a balloon shape. The curve indicated by the dot-and-dash line extending from the black circle at the age of 9 to the right side is a right eye prediction curve 72, which shows the change mode of the ocular axial length of the right eye in the future. The curve indicated by the chain double-dashed line extending from the black square at the age of 9 to the right side is a left eye prediction curve 74, which shows the change mode of the ocular axial length of the left eye. On the intersections of the right eye prediction curve 72 and the left eye prediction curve 74 with the vertical axis at 10 years old, the respective predicted ocular refractive power values (-2.5 D) are indicated. After the alert process (S26-6) shown in FIG. 9, a future visual image acquisition process (S26-7) is performed to acquire a future visual image based on the prediction data of the change mode of the ocular axial length in the future. At the subsequent S26-8, a future visual image display process is performed to display the future visual image acquired at S26-6 on the display part 20 of the user terminal device 12. By so doing, when the intersection of the right eye prediction curve 72 or the left eye prediction curve 74 with the vertical axis at 10 years old is clicked, the future visual image is displayed on the graph. Although it is often difficult for infants and children to understand what "eyesight deteriorating" means even if they are alerted at the alert process (S26-6), the future visual image display process allows them to confirm the actual visual image thereby prompting the users to voluntarily participate in the myopia progression control treatment. The visual image can be acquired by using a "site to experience how myopia looks (Eye Sim)" or other means. It may also be acquired through computing in cooperation with the external software interfaced by the API (application programming interface).

Like the example shown in FIG. 8, in the case where the measurement acquisition process (S21) is performed by the user a plurality of times with a time interval, at S26-9 of the flowchart shown in FIG. 9, it is acceptable to include a change rate acquisition process obtaining the rate of change on the time axis for the plurality of the measurements of the ocular axial length (at the ages of 7, 8 and 9). By acquiring the "rate of change" for each measurement of the ocular axial length, the change in the ocular axial length can be evaluated with the time axis, and it is possible to more accurately grasp the tendency toward myopia and to accurately grasp the effect of the myopia progression control treatment and the like. The rate of change for the plurality of pieces of prediction data (at the ages of 7, 8 and 9) may also be acquired. This allows comparison of the rates of change between the prediction data and the measurements to even more accurately grasp the tendency toward myopia and the degree of effect of the myopia progression control treatment.

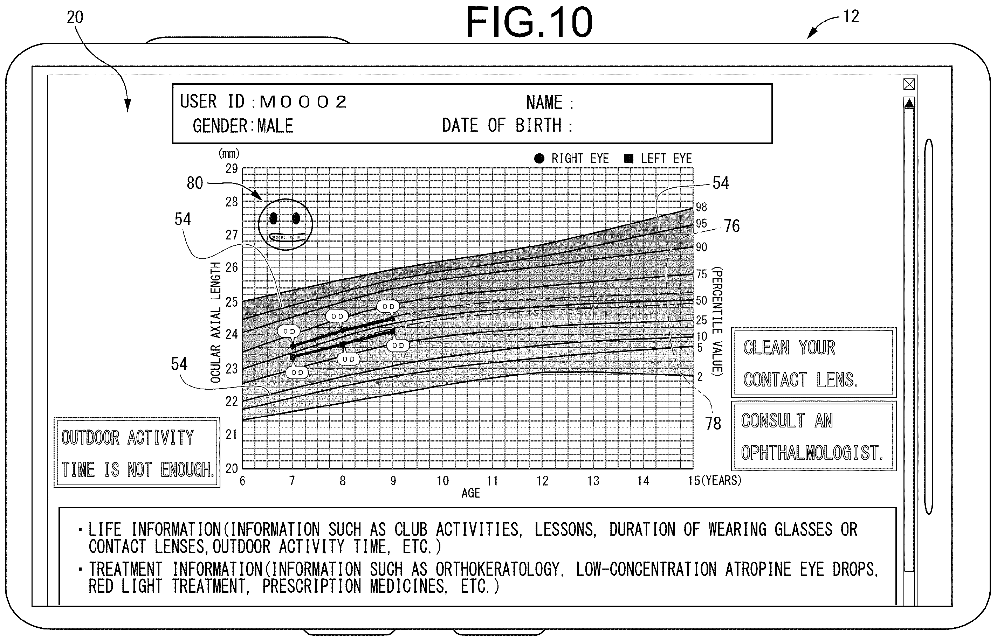

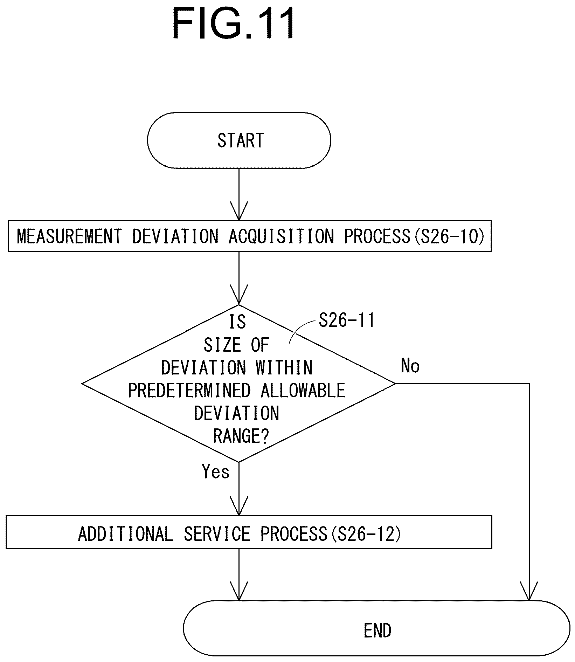

Next, in the example shown in FIG. 10, the measurement acquisition process (S21) is performed three times with a time interval, including the times when the user, a 9-year-old boy, was 7 and 8 years old, and the measurements of the ocular axial length taken at the past three times are displayed on the graph together with the prediction data. At the ages of 7 and 8, the left and right ocular axial lengths were both near the 50th percentile, and it can be easily confirmed from the ocular refractive power values (0D) shown nearby that he had emmetropic eyes. Still at the age of 9, the left and right ocular axial lengths are both near the 50th percentile, and the sizes of separation from the right eye prediction curve 76 and the left eye prediction curve 78 at age 8, which are indicated by the dot-and-dash line and the chain double-dashed line, respectively, are within the allowable deviation range (e.g., within 0.1 mm). In the example shown in FIG. 10, the prediction data display process (S26) shown in FIG. 3 may include the steps shown in FIG. 11.

As shown in FIG. 11, in the case where the measurement acquisition process (S21) is performed a plurality of times with a time interval, at S26-10, the measurement deviation acquisition process is performed to obtain the deviation between the predicted ocular axial length at the age of 9 in the prediction data (the right eye prediction curve 76 / the left eye prediction curve 78), which was acquired by the prediction data acquisition process previously (at age 8), and the measurement of the ocular axial length, which is acquired by the measurement acquisition process (S21) this time (at age 9). Then, at S26-11, it is checked whether or not the size of the deviation acquired by the measurement deviation acquisition process (S26-10) is within the predetermined allowable deviation range (e.g., within 0.1 mm). In the example in FIG. 10, the measurement of the ocular axial length at the age of 9 is within 0.1 mm from the ocular axial length at the age of 9 in the prediction data, so that the determination at S26-11 is YES. If the determination at S26-11 is YES, an additional service process is performed to provide an additional service at S26-12. The additional service process (S26-12) can adopt, for example, a character display or an animation display on the graph in FIG. 10, awarding of points, and the like (see a character display 80 in FIG. 10). This can increase motivation of the users (the age group of infants and children) for myopia prevention and myopia progression control treatment.

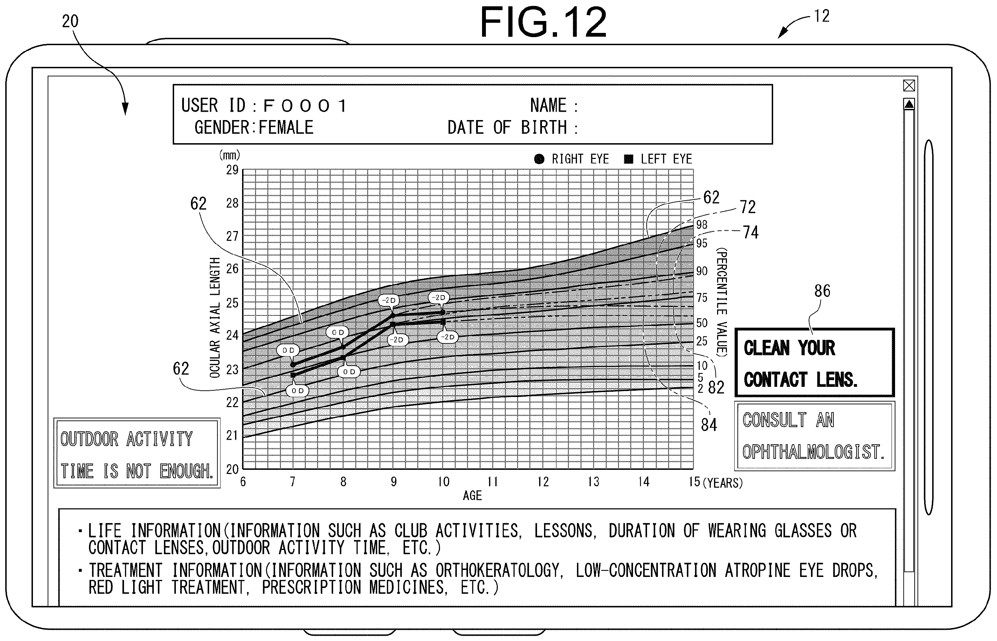

Next, the example shown in FIG. 12 shows, for example, the state where the girl illustrated in FIG. 8 above was determined to have myopia progressing at the age of 9, and then underwent myopia progression control treatment, and measured her ocular axial length (by the measurement acquisition process (S21)) at the age of 10, in which the respective ocular axial lengths in the right and left eyes at the measurement times of 7 to 10 years old are shown. In this example, the myopia progression control treatment is assumed to be orthokeratology. As shown in FIG. 12, as a result of continued myopia progression control treatment (orthokeratology), the ocular axial length in each of the right and left eyes has not changed much compared to that at the age of 9. That is, as shown in FIG. 8, without myopia progression control treatment, according to the right eye prediction curve 72 and the left eye prediction curve 74 at the age of 9, myopia would have progressed to an ocular refractive power value of about -2.5 D at the age of 10, but with myopia progression control treatment (orthokeratology), extension of the ocular axial length is controlled (namely, myopia progression is controlled), resulting in the same level of ocular refractive power value (-2D) as at the age of 9. Accordingly, a right eye prediction curve 82 and a left eye prediction curve 84 at the age of 10 are different from those at the age of 9, indicating that myopia is controlled to some extent by continuing myopia progression control treatment (orthokeratology), which are the prediction data reflecting the effect of myopia progression control treatment.

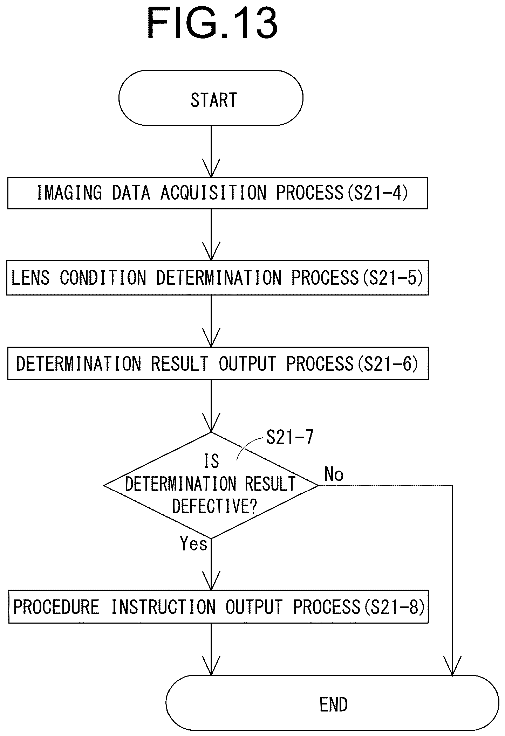

In such orthokeratology, contact lenses for myopia progression control treatment are used for treatment. Here, in the example shown in FIG. 12, the measurement acquisition process (S21) shown in FIG. 3 may include the steps shown in FIG. 13. Specifically, an imaging data acquisition process (S21-4) acquiring lens imaging data for the contact lens for myopia progression control treatment, a lens condition determination process (S21-5) determining the condition of the contact lens for myopia progression control treatment from the lens imaging data acquired by the imaging data acquisition process, and a determination result output process (S21-6) outputting the determination result of the lens condition acquired by the lens condition determination process (S21-5) may be further included. Then, at S21-7, it is checked whether or not the determination result outputted by the determination result output process (S21-6) is defective. If it is defective (S21-7 = Yes), a procedure instruction output process (S21-8) may be further included to instruct the user about a necessary procedure.

Specifically, the contact lens for myopia progression control treatment is photographed while the user is wearing the contact lens. This photographing of the lens may be performed daily or every predetermined number of days. Besides, the imaging data acquisition process (S21-4) mentioned above may be performed separately from the measurement acquisition process (S21), i.e., when the user is not wearing the contact lens. The lens imaging data acquired by this process is analyzed for the degree of contamination caused by protein, etc. accumulated in the lens using known image processing software or the like. If the degree of contamination in the lens exceeds a predetermined amount, that is, if the determination result outputted at the determination result output process (S21-6) is defective, as shown in FIG. 12, a lens cleaning alert 86 is displayed, for example, on the display part 20 of the user terminal device 12, which prompts the user to clean the contact lens. This allows the user to grasp the contamination of the contact lens for myopia progression control treatment and to use the lens in a clean condition by cleaning it. Here, the specific mode of the lens cleaning alert 86 is not limited. A message prompting the user to clean the lens may be displayed as shown in FIG. 12, or a specific part of the display part 20 may be blinked, or specific music, etc. may be played. Additionally, the determination result of the lens condition and the lens cleaning alert 86 may be notified not only to the user (the patient himself/herself), but also to the user's guardian or others, for example, through an application such as an e-mail.

Indeed, the procedure instructions provided to the user by the procedure instruction output process (S21-8) may include not only lens cleaning, but also lens replacement, etc. That is, if the amount of contamination does not fall below the predetermined amount even after the contact lens has been cleaned to be photographed and analyzed again, or if the expiration date of the contact lenses has passed, etc., instructions for lens replacement instead of lens cleaning may be outputted. Therefore, in addition to the lens cleaning alert 86, the display part 20 of the user terminal device 12 may be provided with a lens replacement alert that prompts the user to replace the lens.

According to the information processing system 10 for myopia management service of the present practical embodiment, the application program 30a is executed on the user terminal device 12. This provides the application program 30a for the user who is the target of myopia prevention and progression control, and directly appeals to the user himself/herself, thereby making it possible to increase user's motivation for myopia prevention and progression control treatment. As a result, a new technical effect is achieved by realizing the information processing system 10 that can provide a myopia management service that is able to realize preventive medical treatment and early myopia progression control treatment targeting ophthalmology, which were not available in the past. In particular, measurements of the ocular axial length, which are important for evaluating the effect of myopia prevention and myopia progression control treatment, are acquired, and the prediction data of the change mode of the ocular axial length in the future is presented as a graphical display overlapped on the percentile curve of the ocular axial length of elementary and junior high school students on the time axis. This makes it possible to help the users understand the necessity for myopia prevention and progression control treatment by using the ocular axial length, which was not very familiar to the users in the past. As a result, it has become possible to realize the application program 30a for the user which is able to provide the myopia management service that can disseminate preventive medical measures for myopia and myopia progression control treatment targeting ophthalmology, and the information processing system 10 including the application program 30a.

Here, the user terminal device 12 implementing the application program 30a can be regarded as a myopia management device used by a user who is a target of the myopia management service. The myopia management device can include the display part 20 displaying the measurements of the ocular axial length specific to the user on a time axis, and a function displaying a plurality of measurements of ocular axial length for the user that are measured with a time interval. Such a myopia management device for a user may display the prediction data of the change mode of the ocular axial length in the future, which are obtained based on the measurement of the ocular axial length of the user, on the display part 20 together with the measurement.

Besides, according to the information processing system 10 of the present practical embodiment, it is possible to advantageously perform an ocular information processing method for a user wherein the user terminal device 12 acquires and stores the measurement of the ocular axial length of the user, and displays the stored measurement of the ocular axial length on the graph on the time axis on the display part 20 of the user terminal device 12. Such an ocular information processing method may acquire and store the prediction data of the change mode of the ocular axial length in the future of the user, which is obtained based on the measurement of the ocular axial length, and may display the stored measurement of the ocular axial length and the stored prediction data of the ocular axial length together on the graph on the display part 20.

### Variations

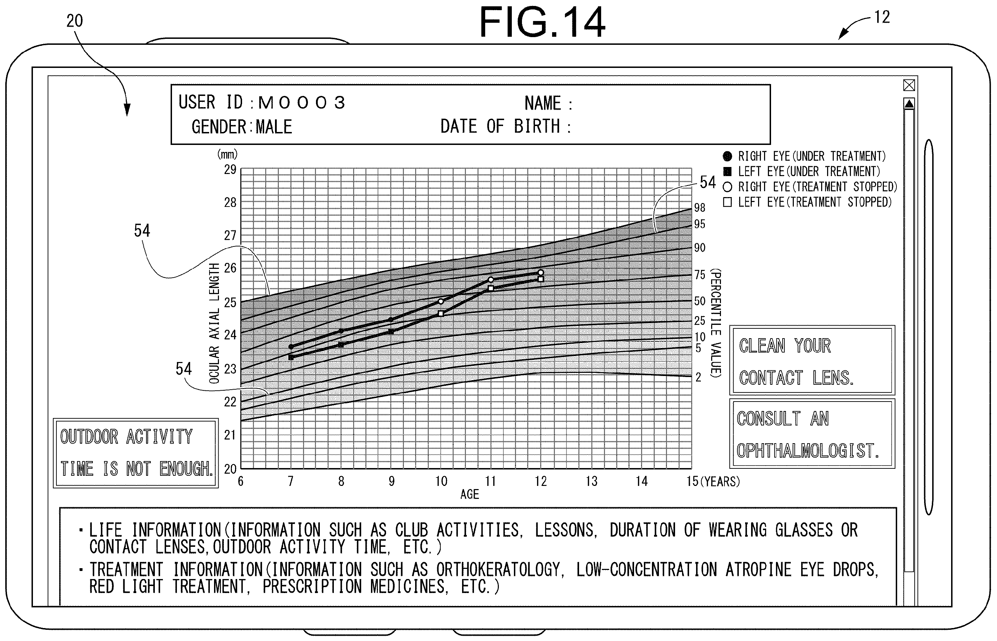

Although the practical embodiment has been described in detail as a specific example of the present disclosure, the present disclosure is not limited by these specific descriptions. Modifications, improvements, etc. to the extent that the object of the present disclosure can be achieved are included in the present disclosure. For example, the following variations of the practical embodiment are also included in the technical scope of the present disclosure.
(1) The preceding practical embodiment shows an example in which, if the user is prescribed myopia progression control treatment, the treatment information acquisition process (S23) is performed to acquire the information of the myopia progression control treatment prescribed to the user. In this case, the practical embodiment may be modified to further include an information transmission process for an ophthalmologist transmitting the measurement acquired by the measurement acquisition process (S21) and the myopia progression control treatment information acquired by the treatment information acquisition process (S23) inclusively to the ophthalmologist terminal device 50 used by the ophthalmologist. This makes it possible to efficiently provide continuous treatment even when the user's ophthalmologist changes due to the user's relocation or the like.
(2) In the preceding practical embodiment, in the graphical displays illustrated in FIGS. 6, 8, 10, and 12, the range of the time axis is from 6 to 15 years old in all cases, but is not limited to the above examples. For example, in the application program 30a, at the prediction data display process (S25) and the measurement display process (S27), it would be acceptable to enable a display mode change process changing the range of the time axis displayed on the display part 20 thereby zooming in and out the display mode. This allows the display to switch between short periods, such as several months or six months, and long periods, such as several years, when the myopia progression control treatment has been performed over a long period of time. By so doing, the necessary information of the change mode can be visually observed in an efficient manner, thereby improving the convenience.
(3) The user who uses the application program, the information processing system for the myopia management service, the myopia management device, and the ocular information processing method according to the present disclosure is not only the patient himself/herself who controls the myopia progression or undergoes the treatment for myopia, but may also be a guardian of the patient.
(4) In the preceding practical embodiment, the prediction data of the change mode of the ocular axial length (e.g., the right eye prediction curve 56 or the left eye prediction curve 58) is shown as a curve, but may be, for example, an approximate straight line to this curve.
(5) As shown in FIG. 14, in the case where the ocular axial length is measured a plurality of times with a time interval by the measurement acquisition process, the measurements of the ocular axial length may include those during treatment of myopia progression control treatment and those during a stop of myopia progression control treatment. In the example in FIG. 14, the ocular axial lengths measured during the treatment of myopia progression control treatment (e.g., orthokeratology) are shown on the graph as black circles and black squares, while the ocular axial lengths measured during the stop of myopia progression control treatment are shown on the graph as white circles and white squares. In this way, the measurements of the ocular axial lengths acquired during the treatment of the myopia progression control treatment and the measurements of the ocular axial lengths acquired during the stop of the myopia progression control treatment are distinguishable on the graph displayed on the display part 20. In the example shown in FIG. 14, it is indicated that the extension of the ocular axial length is controlled to some extent during the treatment of the myopia progression control treatment, while the ocular axial length extends significantly during the stop of the myopia progression control treatment. Thus, it is understood that the myopia progression control treatment is effective in controlling myopia progression. Then, when the ocular axial length is measured after a predetermined period of time has passed since the myopia progression control treatment is stopped, and if it is confirmed that the extension of the ocular axial length, i.e., myopia, has progressed, the myopia progression control treatment may be restarted. Alternatively, if no significant extension in the ocular axial length can be confirmed when the ocular axial length is measured after a predetermined period of time has passed since the myopia progression control treatment is stopped, the treatment may be kept stopped. After the treatment is restarted, if it is confirmed that the extension of the ocular axial length has been controlled to some extent, the treatment may be stopped again.

In the example shown in FIG. 14, the ocular axial length measured during treatment with orthokeratology is indicated by the black circles and the black squares. However, the shape, color, etc. of the points may be different depending on the treatment method (e.g., eye drops, orthokeratology, red light treatment, glasses, etc.). By so doing, from the graph, it is possible to easily grasp what type of treatment has been performed or what type of treatment is being continued, and what type of treatment has been effective to what extent. Besides, detailed information may also be displayed by tapping on points in the graph. For example, the type of treatment method mentioned above, as well as the ocular refractive power value (D) at each point and the like, may be displayed.

## Claims

1. An application program (30a) for a user performing processes in a user terminal device (12) including a display part (20), the user terminal device (12) being used by the user who is a target of a myopia management service, the processes comprising:
a measurement acquisition process acquiring at least one measurement of an ocular axial length of the user; and
a measurement display process displaying the measurement acquired by the measurement acquisition process on a graph on a time axis on the display part (20).

2. The application program (30a) according to claim 1, wherein
the measurement acquisition process is performed a plurality of times with a time interval, and the at least one measurement of the ocular axial length comprises a plurality of measurements of the ocular axial length, and
the measurement display process displays the plurality of measurements of the ocular axial length acquired by the measurement acquisition process performed the plurality of times on the graph on the display part (20).

3. The application program (30a) according to claim 1 or 2, wherein the processes further comprise:
a prediction data acquisition process acquiring prediction data of a change mode of an ocular axial length in a future obtained based on the measurement acquired by the measurement acquisition process; and
a prediction data display process displaying the prediction data acquired by the prediction data acquisition process on the graph on the time axis on the display part (20) together with the measurement acquired by the measurement acquisition process.

4. The application program (30a) according to claim 3, wherein the prediction data acquired by the prediction data acquisition process includes prediction data of a change mode of an ocular axial length of an emmetropic eye.

5. The application program (30a) according to claim 3 or 4, wherein the prediction data acquired by the prediction data acquisition process includes prediction data reflecting an effect of myopia progression control treatment.

6. The application program (30a) according to any one of claims 3-5, wherein the processes further comprise:
a measurement deviation acquisition process obtaining a deviation between the prediction data acquired by the prediction data acquisition process and the measurement acquired by the measurement acquisition process; and
an additional service process providing an additional service if a size of the deviation obtained by the measurement deviation acquisition process is within a predetermined allowable deviation range.

7. The application program (30a) according to any one of claims 1-6, wherein the processes further comprise an ocular refractive power value acquisition process acquiring an ocular refractive power value of the user.

8. The application program (30a) according to any one of claims 3-6, or claim 7 when dependent on claim 3, wherein in the prediction data display process, the prediction data is displayed as a band-shaped area extending on the time axis on the display part (20).

9. The application program (30a) according to any one of claims 1-8, wherein
the measurement acquisition process is performed a plurality of times with a time interval, and the at least one measurement of the ocular axial length comprises a plurality of measurements of the ocular axial length, and
the processes further comprise a change rate acquisition process obtaining a rate of change on the time axis for the plurality of measurements of the ocular axial length acquired by the measurement acquisition process performed the plurality of times.

10. The application program (30a) according to any one of claims 1-9, wherein the processes further comprise:
an imaging data acquisition process acquiring lens imaging data for a contact lens provided to the user for myopia progression control treatment;
a lens condition determination process determining a condition of the contact lens from the lens imaging data acquired by the imaging data acquisition process; and
a determination result output process outputting a determination result of the condition of the contact lens acquired by the lens condition determination process.

11. The application program (30a) according to any one of claims 1-10, wherein the processes further comprise:
a treatment information acquisition process acquiring information of myopia progression control treatment prescribed to the user; and
an information transmission process for an ophthalmologist transmitting the measurement acquired by the measurement acquisition process and the information of the myopia progression control treatment acquired by the treatment information acquisition process inclusively to an ophthalmologist terminal device (50) used by the ophthalmologist.

12. The application program (30a) according to any one of claims 3-6, or any one of claims 7 and 9-11 when dependent on claim 3, wherein
the prediction data display process and the measurement display process further comprise a display mode change process changing a range of the time axis displayed on the display part (20) to zoom in and out a display mode.

13. The application program (30a) according to any one of claims 1-12, wherein the processes further comprise:
a life information acquisition process acquiring a life situation data of the user; and
a life information display process displaying the life situation data acquired by the life information acquisition process together with time axis information on the display part (20).

14. The application program (30a) according to any one of claims 1-13, wherein the processes further comprise:
a member information acquisition process acquiring member registration information of the user; and
a member information display process displaying the member registration information of the user acquired by the member information acquisition process on the display part (20).

15. The application program (30a) according to claim 2, or any one of claims 3-14 when dependent on claim 2, wherein
the plurality of measurements of the ocular axial length acquired by the measurement acquisition process performed the plurality of times include the measurement during treatment of myopia progression control treatment and the measurement during a stop of the myopia progression control treatment, and
the measurement of the ocular axial length acquired during the treatment of the myopia progression control treatment and the measurement of the ocular axial length acquired during the stop of the myopia progression control treatment that are displayed on the display part (20) by the measurement display process are distinguishable on the graph.
